# EUROPÄISCHE PATENTSCHRIFT

(11) **EP 2 859 879 B1**
(45) Veröffentlichungstag und Bekanntmachung des Hinweises auf die Patenterteilung: **20.12.2017**
(21) Anmeldenummer: 14159781.5
(22) Anmeldetag: 14.03.2014
(51) Int. Cl.: A61K 8/34, A61K 8/49, A61Q 11/00

(54) **Mund- und Zahnpflege- und -reinigungsmittel gegen Halitosis**
Oral and tooth care and cleaning agents to prevent halitosis
Produit de nettoyage et de soin de la bouche et des dents contre l'halitose

(30) Priorität: 03.07.2013 DE 102013212999
(43) Veröffentlichungstag der Anmeldung: 15.04.2015
(73) Patentinhaber: Henkel AG & Co. KGaA, 40589 Düsseldorf (DE)
(72) Erfinder: Miehlich, Kristin, 42119 Wuppertal (DE); Arians, Daniela, 45239 Essen (DE); Welß, Thomas, 40591 Düsseldorf (DE); Jassoy, Claudia, 40237 Düsseldorf (DE)

(56) Entgegenhaltungen:
- EP-B1- 1 793 796
- US-A1- 2002 054 859
- US-A1- 2005 201 953
- INESS BETTAIEB ET AL: "Essential oils and fatty acids composition of Tunisian and Indian cumin (Cuminum cyminum L.) seeds: a comparative study", JOURNAL OF THE SCIENCE OF FOOD AND AGRICULTURE, Bd. 91, Nr. 11, 30. August 2011 (2011-08-30), Seiten 2100-2107, XP055146052, ISSN: 0022-5142, DOI: 10.1002/jsfa.4513
- CHALCHAT J C ET AL: "Comparative essential oil composition of flowers, leavesand stems of basil (Ocimum basilicum L.) used as herb", FOOD CHEMISTRY, Bd. 110, Nr. 2, 15. September 2008 (2008-09-15), Seiten 501-503, XP022614730, ELSEVIER LTD, NL ISSN: 0308-8146, DOI: 10.1016/J.FOODCHEM.2008.02.018 [gefunden am 2008-02-16]
- PIETRO LO CANTORE ET AL: "Antibacterial Activity of Coriandrum sativum L. and Foeniculum vulgare Miller Var. vulgare (Miller) Essential Oils", JOURNAL OF AGRICULTURAL AND FOOD CHEMISTRY, Bd. 52, Nr. 26, 1. Dezember 2004 (2004-12-01), Seiten 7862-7866, XP055146080, ISSN: 0021-8561, DOI: 10.1021/jf0493122
- DATABASE CAPLUS [Online] CHEMICAL ABSTRACTS SERVICE, COLUMBUS, OHIO, US; 31. Dezember 2010 (2010-12-31), Marzec: "Evaluation of essential oil content", XP002731084, Database accession no. 2011:92349
- BÁRBARA TEIXEIRA ET AL: "Chemical composition and antibacterial and antioxidant properties of commercial essential oils", INDUSTRIAL CROPS AND PRODUCTS, Bd. 43, 1. Mai 2013 (2013-05-01), Seiten 587-595, XP055146373, ISSN: 0926-6690, DOI: 10.1016/j.indcrop.2012.07.069
- Vaidehi Bhave: "Home Remedies - Cumin seeds", Tips4.net , 21. September 2009 (2009-09-21), XP002731085, Gefunden im Internet: URL:http://www.tips4.net/2009/09/home-reme dies-of-cumin-seeds.html [gefunden am 2014-10-13]
- Jeff Cohen: "5 Herbs Can Stop Halitosis (Bad Breath)", KissMeGoodnight.com , 31. Dezember 2006 (2006-12-31), XP002731086, Gefunden im Internet: URL:http://www.kissmegoodnight.com/bad-bre ath/5-herbs-for-bad-breath.shtml [gefunden am 2014-10-13]
- Eleonore Hohenberger: "Gibt es ein Mittel gegen Mundgeruch?", BKK24 Länger besser leben , 14. Oktober 2014 (2014-10-14), XP002731087, Gefunden im Internet: URL:http://www.bkk24.de/typo3/index.php?id =1311 [gefunden am 2014-10-14]
- Anonymous: "Bad Breath", Health911 , 31. Dezember 2012 (2012-12-31), XP002731088, Gefunden im Internet: URL:http://www.health911.com/bad_breath [gefunden am 2014-10-14]
- DATABASE GNPD [Online] MINTEL; 1. Oktober 2013 (2013-10-01), "Ipsa Labs Arodent Ayurvedic Gum & Dental Paste", XP002731089, Database accession no. 2178747
- DATABASE GNPD [Online] MINTEL; "Natural Toothpaste", XP002731090, Database accession no. 25064
- DATABASE GNPD [Online] MINTEL; 1. Mai 2008 (2008-05-01), "Prodent Whitener Natural Fresh Toothpaste", XP002731091, Database accession no. 918974
- DATABASE GNPD [Online] MINTEL; 1. September 2010 (2010-09-01), "All-Natural Soothing Tooth Gel with CoQ10", XP002731092, Database accession no. 1392341
- DATABASE GNPD [Online] MINTEL; 1. März 2010 (2010-03-01), "Fluoride Toothpaste", XP002731093, Database accession no. 1268309
- ISENGARD HEINZ-DIETER; MATHLOUTHI MOHAMED: "Editorial Board / Aims & Scope", FOOD CHEMISTRY, Bd. 112, Nr. 3, 1. Februar 2009 (2009-02-01), Seite IFC, XP023905363, ELSEVIER LTD, NL ISSN: 0308-8146, DOI: 10.1016/S0308-8146(08)00949-7 [gefunden am 2008-08-14]
- BASLAS R K ET AL: "Chemical examination of essential oils from plants of genus Anethum (Umbelliferae)-oil of seeds of Anethum graveolens. I", FLAVOUR INDUSTRY, UNITED TRADE PRESS, LONDON, GB, vol. 2, no. 4, 1 January 1971 (1971-01-01) , pages 241-245, XP009190390, ISSN: 0015-3532
- Jeet Gurinder ET AL: "Bioactive potential of Anethum graveolens, Foeniculum vulgare and Trachyspermum ammi belonging to the family Umbelliferae -Current status", Journal of Medicinal Plants Research, 18 January 2010 (2010-01-18), pages 87-94, XP55278366, DOI: 10.5897/JMPR09.018 Retrieved from the Internet: URL:http://www.academicjournals.org/articl e/article1380375140_Kaur and Arora.pdf

## Beschreibung

Die Erfindung betrifft Zubereitungen zur Mund- und Zahnpflege- und -reinigung, die aufgrund von speziellen Inhaltsstoffen die Verhinderung oder Behandlung von Mundgeruch bzw. Halitosis, Gingivitis oder Parodontitis ermöglichen.

Zahnreinigungsmittel sind in verschiedenen Formen auf dem Markt und dienen in erster Linie der Reinigung der Zahnoberfläche und der Vorbeugung von Zahn- und Zahnfleischerkrankungen. Sie enthalten üblicherweise eine Kombination aus Poliermitteln, Feuchthaltemitteln, Tensiden, Bindemitteln, Aromastoffen und fluoridhaltigen sowie antimikrobiellen Wirkstoffen. Neben Zahnpulvern, die wegen ihrer erhöhten Abrasivität eine untergeordnete Rolle spielen, werden Zahnreinigungsmittel vor allem in Pasten-, Creme- und transluzenter oder transparenter Gelform angeboten. In den letzten Jahren haben auch Liquid- oder Flüssigzahncremes und Mundwässer zunehmend an Bedeutung gewonnen.

Neben der Reinigung der Zähne erwartet der Verbraucher von den gattungsgemäßen Produkten auch eine Pflege der Zähne und der Mundhöhle. So sind insbesondere ein "sauberes" Gefühl, d.h. eine glatte und glänzende Zahnoberfläche sowie ein frisches Gefühl im Mund wesentliche Aspekte für den Kaufanreiz von Zubereitungen zur Mund- und Zahnpflege- und -reinigung. Ein erfolgreiches Mittel der gattungsgemäßen Art sollte daher die Zähne gründlich reinigen, ohne den Zahn oder die Zahnoberfläche zu schädigen und gleichzeitig Mundgeruch verringern und/oder verhindern.

Mundgeruch, auch Halitosis oder "Foetor ex ore" genannt, kann verschiedene Ursachen haben. Bei gesunden Menschen wird Mundgeruch jedoch in den meisten Fällen durch Bakterien im Mund- bzw. Rachenraum hervorgerufen, die Körperzellen oder Nahrungsreste verstoffwechseln, wobei als Abbauprodukte von Proteinen u.a. flüchtige Schwefelverbindungen ("volatile sulfur compounds", VSC) entstehen, die für den üblen Geruch verantwortlich gemacht werden.

Diese Schwefelverbindungen sind insbesondere: H₂S = Schwefelwasserstoff (zu etwa 30 %), CH₃-S-H = Methylmercaptan (zu etwa 60 %) und CH₃-S-CH₃ = Dimethylsulfid (zu etwa 10 %).

Die flüchtigen Schwefelverbindungen sind teilweise sehr aggressiv und können das Zahnfleisch und

die Mundschleimhaut schädigen. So ist beispielsweise bekannt, dass Schwefelwasserstoff und Methylmercaptan die Durchlässigkeit der Mundschleimhaut erhöhen und so Zahnfleischerkrankungen Vorschub leisten können.

Zahnfleischerkrankungen stellen einen hohen Risikofaktor für die Gesundheit dar. Nach Untersuchungen der Weltgesundheitsorganisation (WHO) leiden über 50 % der erwachsenen Bundesbürger an dringend behandlungsbedürftigen Parodontopathien.

Zahnfleischerkrankungen werden im Volksmund oft "Parodontose" genannt. Dieser Ausdruck ist nicht korrekt, da die Endung "-ose" einen normalen, altersbedingten Rückgang eines Organs beschreibt. Richtig sind die Ausdrücke "Parodontitis" oder auch "entzündliche Parodontopathie". Sie ist neben der Karies die weit verbreiteste Erkrankung der Mundhöhle und tritt hauptsächlich bei Erwachsenen auf.

Unter Parodontitis versteht man eine durch Bakterien hervorgerufene entzündliche Veraenderung des den Zahn umgebenden Gewebes, besonders des Kieferknochens. Eine Parodontitis (= mit Beteiligung der Zahnfleischtaschen und des Knochens) entwickelt sich immer aus einer Zahnfleischentzündung (Gingivitis) und befällt zuerst die der Reinigung am schlechtesten zugänglichen Zahnzwischenräume. Die sich in der Zahnfleischtasche befindlichen Bakterien wirken durch ihre Stoffwechselprodukte, insbesondere durch die von Ihnen produzierten VSC, destruktiv auf Zahnfleisch, Zahnsubstanz und Knochen ein und verstärken so ständig die bereits bestehende Parodontitis.

Dieses gesundheitliche Problem ist sehr weit verbreitet: Untersuchungen zeigen, dass etwa ab dem 35. Lebensjahr mehr Zähne durch Parodontopathien (Zahnfleischerkrankungen) als durch Karies verloren gehen.

Es hat nicht an Versuchen gefehlt, Mundgeruch zu bekämpfen, und eine Vielzahl von Agentien wird zu diesem Zweck in Zubereitungen zur Mund- und Zahnpflege und -reinigung eingesetzt. Als antibakterielle Mittel finden beispielsweise Triclosan, Zinnsalze oder Chlorhexidin Verwendung.

Der Einsatz dieser Produkte kann jedoch in Einzelfällen mit Nachteilen verbunden sein, die teils geschmacklicher Natur sind, teils ästhetische Gründe haben, da das Produkt und/oder die Zähne durch übermäßigen Gebrauch der Agentien verfärbt werden können.

Es besteht daher nach wie vor das Bedürfnis, Halitosis zu bekämpfen und Wirkstoffe oder Wirkstoffkombinationen dafür bereitzustellen, die frei von den genannten Nachteilen sind. Aromazubereitungen gegen Mundgeruch und schlechten Atem werden beispielsweise in der EP 1 793 796 B1 offenbart. Dort wird die Kombination von Eugenolacetat und Isoeugenolmethylether als besonders wirksam offenbart.

In den Marktprodukten "Ipsa Labs Arodent Ayurvedic Gum & Dental Paste" (Mintel-Nr. 2178747), "Natural Toothpaste" (Mintel-Nr. 25064), "Prodent Whitener Natural Fresh Toothpaste" (Mintel-Nr. 918974), "All Natural Soothing Tooth Gel witt CoQ10" (Mintel-Nr. 1392341) und "Fluoride Toothpaste" (Mintel-Nr. 1268309) wird Kümmelextrakt bzw. Fenchelextrakt bzw. Peterseilien- und Nelkenextrakt eingesetzt. Alle diese Extrakte enthalten Apiol bzw. Dillapiol.

In der US2002/054859 A1 wird eine Kombination von (iso-)Apiol und Isoeugenol offenbart US2005/0201953 befasst sich mit Bekämpfung des Mundgeruchs. Die Aufgabe der vorliegenden Erfindung bestand darin, Zubereitungen zur Mund- und Zahnpflege und -reinigung bereitzustellen, die Mundgeruch wirksam bekämpfen und gegen Gingivitis und Parodontitis wirksam sind. Dabei sollten die Nachteile des Einsatzes von Triclosan, Zinnsalzen oder Chlorhexidin, insbesondere die Verfärbung von Zahnoberflächen, vermieden werden können.

Es wurde nun gefunden, dass sich Mund und Zahnpflege- und -reinigungsmittel mit gesteigerter Wirkung gegen Halitosis formulieren lassen, wenn diese mindestens zwei bestimmte Verbindungen enthalten. Diese Zusammensetzungen eignen sich darüber hinaus auch zur Behandlung von Gingivitis oder Parodontitis und sind herkömmlichen Mitteln überlegen. Vorzugsweise kann die Kombination durch weitere Inhaltsstoffe, insbesondere Zinksalze, in ihrer Wirkung gesteigert werden.

Gegenstand der vorliegenden Erfindung ist in einer ersten Ausführungsform ein Mund- und Zahnpflege- und -reinigungsmittel, enthaltend - bezogen auf sein Gewicht -
a) 0,00001 bis 1 Gew.-% Verbindung(en) aus der Gruppe
   a. 4,7-Dimethoxy-5-(prop-2-enyl)-2H(1,3-benzodioxol) und/oder
   b. 4,5-Dimethoxy-6-(prop-2-enyl)-2H(1,3-benzodioxol) und/oder
   c. Mischungen aus diesen;
b) 0,001 bis 1 Gew.-% Verbindung(en) aus der Gruppe
   a. 2-methoxy-4-(prop-2-enyl)-phenol und/oder
   b. 2-methoxy-4-(prop-1-enyl)-phenol und/oder
   c. Mischungen aus diesen.
a) 10 bis 50 Gew mehrwertige(n) Alkohol(e) aus der Gruppe Sorbit und/oder Glycerin und/oder 1,2-Propylenglycol oder deren Mischungen
mit der Maßgabe, daß das Mund- und Zahnpflege- und -reinigungsmittel 0,05 bis 0,4 Gew.-%, 2-methoxy-4-(prop-1-enyl)-phenol 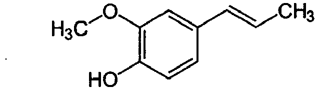 enthält.

Mund- und Zahnpflegemittel sowie Mund- und Zahnreinigungsmittel im Sinne der Erfindung sind Mund- und Zahnpulver, Mund- und Zahnpasten, flüssige Mund- und Zahncremes sowie Mund- und Zahngele. Bevorzugt geeignet sind Zahnpasten und flüssige Zahnreinigungsmittel. Hierzu können die Mund- und Zahnpflege- und reinigungsmittel z.B. in Form von Zahnpasten, flüssigen Zahncremes, Zahnpulvern, Mundwässern oder gegebenenfalls auch als Kaumasse, z. B. als Kaugummi, vorliegen. Bevorzugt liegen sie jedoch als mehr oder weniger fließfähige oder plastische Zahnpasten vor, wie sie zur Reinigung der Zähne unter Einsatz einer Zahnbürste verwendet werden.

Die erfindungsgemäßen Mund- und Zahnpflege- und -reinigungsmittel enthalten als wesentlichen Inhaltsstoff 4,7-Dimethoxy-5-(prop-2-enyl)-2H(1,3-benzodioxol) und/oder 4,5-Dimethoxy-6-(prop- 2-enyl)-2H(1,3-benzodioxol) in einer Gesamtmenge von 0,00001 bis 1 Gew.-%, jeweils bezogen auf das erfndungsgemäße Mund- und Zahnpflege- und -reinigungsmittel. Erfindungsgemäße Mittel können demnach ausschließlich 4,7-Dimethoxy-5-(prop-2-enyl)-2H(1,3-benzodioxol) oder ausschließlich 4,5-Dimethoxy-6-(prop-2-enyl)-2H(1,3-benzodioxol) oder eine Mischung aus beiden Substanzen enthalten. Der Einsatz von 4,7-Dimethoxy-5-(prop-2-enyl)-2H(1,3-benzodioxol) 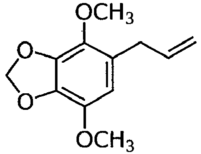 hat sich dabei als besonders wirksam in der erfindungsgemäßen Kombination erwiesen, so dass bevorzugte Mund- und Zahnpflege- und -reinigungsmittel dadurch gekennzeichnet sind, dass sie 0,000025 bis 0,5 Gew.-%, vorzugsweise 0,00005 bis 0,25 Gew.-%, besonders bevorzugt 0,0001 bis 0,1 Gew.-%, weiter bevorzugt 0,0005 bis 0,05 Gew.-% und insbesondere 0,001 bis 0,01 Gew.-% 4,7-Dimethoxy-5-(prop-2-enyl)-2H(1,3-benzodioxol) enthalten.

Weitere bevorzugte Mund- und Zahnpflege- und -reinigungsmittel enthalten 0,000025 bis 0,5 Gew.-%, vorzugsweise 0,00005 bis 0,25 Gew.-%, besonders bevorzugt 0,0001 bis 0,1 Gew.-%, weiter bevorzugt 0,0005 bis 0,05 Gew.-% und insbesondere 0,001 bis 0,01 Gew.-% 4,5-Dimethoxy-6-(prop- 2-enyl)-2H(1,3-benzodioxol).

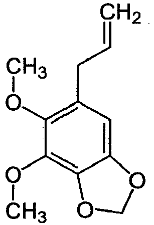

Weitere bevorzugte Mund- und Zahnpflege- und -reinigungsmittel enthalten 0,00002 bis 0,5 Gew.-%, vorzugsweise 0,00004 bis 0,25 Gew.-%, besonders bevorzugt 0,0001 bis 0,1 Gew.-%, weiter bevorzugt 0,00025 bis 0,05 Gew.-% und insbesondere 0,001 bis 0,01 Gew.-% 4,7-Dimethoxy-5-(prop-2-enyl)-2H(1,3-benzodioxol) und 0,00002 bis 0,5 Gew.-%, vorzugsweise 0,00004 bis 0,25 Gew.-%, besonders bevorzugt 0,0001 bis 0,1 Gew.-%, weiter bevorzugt 0,00025 bis 0,05 Gew.-% und insbesondere 0,001 bis 0,01 Gew.-% 4,5-Dimethoxy-6-(prop- 2-enyl)-2H(1,3-benzodioxol).

Als zweiten wesentlichen Inhaltsstoff enthalten die erfindungsgemäßen Mund- und Zahnpflege- und - reinigungsmittel 2-methoxy-4-(prop-2-enyl)-phenol und/oder 2-methoxy-4-(prop-1-enyl)-phenol in einer Gesamtmenge von 0,001 bis 1 Gew.-%, jeweils bezogen auf das erfndungsgemäße Mund- und Zahnpflege- und -reinigungsmittel, mit der Maßgabe, daß das Mund- und Zahnpflege- und - reinigungsmittel 0,05 bis 0,4 Gew.-%, 2-methoxy-4-(prop-1-enyl)-phenol 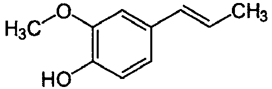 enthält.

Erfindungsgemäße Mittel können ausschließlich 2-methoxy-4-(prop-1-enyl)-phenol oder eine Mischung aus beiden Substanzen enthalten.

Der Einsatz von 2-methoxy-4-(prop-2-enyl)-phenol 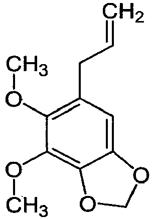 hat sich dabei als besonders wirksam in der erfindungsgemäßen Kombination erwiesen, so dass bevorzugte Mund- und Zahnpflege- und -reinigungsmittel dadurch gekennzeichnet sind, dass sie 0,0025 bis 0,5 Gew.-%, vorzugsweise 0,05 bis 0,4 Gew.-%, besonders bevorzugt 0,01 bis 0,3 Gew.-%, weiter bevorzugt 0,05 bis 0,2 Gew.-% und insbesondere 0,075 bis 0,1 Gew.-% 2-methoxy-4-(prop-2-enyl)-phenol enthalten.

Weitere bevorzugte Mund- und Zahnpflege- und -reinigungsmittel enthalten 0,05 bis 0,2 Gew.-% und insbesondere 0,075 bis 0,1 Gew.-% 2-methoxy-4-(prop-1-enyl)-phenol

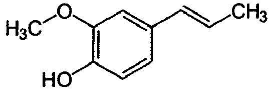

Weitere bevorzugte Mund- und Zahnpflege- und -reinigungsmittel enthalten 0,001 bis 0,4 Gew.-%, vorzugsweise 0,005 bis 0,3 Gew.-%, besonders bevorzugt 0,01 bis 0,25 Gew.-%, weiter bevorzugt 0,05 bis 0,15 Gew.-% und insbesondere 0,05 bis 0,1 Gew.-% 2-methoxy-4-(prop-2-enyl)-phenol und 0,005 bis 0,3 Gew.-%, besonders bevorzugt 0,01 bis 0,25 Gew.-%, weiter bevorzugt 0,05 bis 0,15 Gew.-% und insbesondere 0,05 bis 0,1 Gew.-% 2-methoxy-4-(prop-1-enyl)-phenol.

Die erfindungsgemäßen Mittel können eine Zweierkombination an Wirkstoffen enthalten:
- (4,7-Dimethoxy-5-(prop-2-enyl)-2H(1,3-benzodioxol) und 2-methoxy-4-(prop-1-enyl)-phenol oder
- 4,5-Dimethoxy-6-(prop- 2-enyl)-2H(1,3-benzodioxol) und 2-methoxy-4-(prop-l-enyl)-phenol oder eine Dreierkombination:
- (4,7-Dimethoxy-5-(prop-2-enyl)-2H(1,3-benzodioxol) und 4,5-Dimethoxy-6-(prop- 2-enyl)-2H(1,3-benzodioxol) und 2-methoxy-4-(prop-1-enyl)-phenol oder
- (4,7-Dimethoxy-5-(prop-2-enyl)-2H(1,3-benzodioxol) und 2-methoxy-4-(prop-2-enyl)-phenol und 2-methoxy-4-(prop-1-enyl)-phenol oder
- (4,5-Dimethoxy-6-(prop-2-enyl)-2H(1,3-benzodioxol) und 2-methoxy-4-(prop-2-enyl)-phenol und 2-methoxy-4-(prop-1-enyl)-phenol
oder die Viererkombination:
- (4,7-Dimethoxy-5-(prop-2-enyl)-2H(1,3-benzodioxol) und (4,5-Dimethoxy-6-(prop-2-enyl)-2H(1,3-benzodioxol) und 2-methoxy-4-(prop-2-enyl)-phenol und 2-methoxy-4-(prop-1-enyl)-phenol.

Ganz besonders bevrozugte Mittel enthalten - jeweils bezogen auf ihr Gewicht-
- 0,00002 bis 0,5 Gew.-%, vorzugsweise 0,00004 bis 0,25 Gew.-%, besonders bevorzugt 0,0001 bis 0,1 Gew.-%, weiter bevorzugt 0,00025 bis 0,05 Gew.-% und insbesondere 0,001 bis 0,01 Gew.-% (4,7-Dimethoxy-5-(prop-2-enyl)-2H(1,3-benzodioxol) und 0,005 bis 0,3 Gew.-%, besonders bevorzugt 0,01 bis 0,25 Gew.-%, weiter bevorzugt 0,05 bis 0,15 Gew.-% und insbesondere 0,05 bis 0,1 Gew.-% 2-methoxy-4-(prop-1-enyl)-phenol oder
- 0,00002 bis 0,5 Gew.-%, vorzugsweise 0,00004 bis 0,25 Gew.-%, besonders bevorzugt 0,0001 bis 0,1 Gew.-%, weiter bevorzugt 0,00025 bis 0,05 Gew.-% und insbesondere 0,001 bis 0,01 Gew.-% 4,5-Dimethoxy-6-(prop- 2-enyl)-2H(1,3-benzodioxol) und 0,005 bis 0,3 Gew.-%, besonders bevorzugt 0,01 bis 0,25 Gew.-%, weiter bevorzugt 0,05 bis 0,15 Gew.-% und insbesondere 0,05 bis 0,1 Gew.-% 2-methoxy-4-(prop-1-enyl)-phenol oder
- 0,00002 bis 0,5 Gew.-%, vorzugsweise 0,00004 bis 0,25 Gew.-%, besonders bevorzugt 0,0001 bis 0,1 Gew.-%, weiter bevorzugt 0,00025 bis 0,05 Gew.-% und insbesondere 0,001 bis 0,01 Gew.-% (4,7-Dimethoxy-5-(prop-2-enyl)-2H(1,3-benzodioxol) und 0,00002 bis 0,5 Gew.-%, vorzugsweise 0,00004 bis 0,25 Gew.-%, besonders bevorzugt 0,0001 bis 0,1 Gew.-%, weiter bevorzugt 0,00025 bis 0,05 Gew.-% und insbesondere 0,001 bis 0,01 Gew.-% 4,5-Dimethoxy-6-(prop-2-enyl)-2H(1,3-benzodioxol) und
   0,005 bis 0,3 Gew.-%, besonders bevorzugt 0,01 bis 0,25 Gew.-%, weiter bevorzugt 0,05 bis 0,15 Gew.-% und insbesondere 0,05 bis 0,1 Gew.-% 2-methoxy-4-(prop-1-enyl)-phenol oder
- 0,00002 bis 0,5 Gew.-%, vorzugsweise 0,00004 bis 0,25 Gew.-%, besonders bevorzugt 0,0001 bis 0,1 Gew.-%, weiter bevorzugt 0,00025 bis 0,05 Gew.-% und insbesondere 0,001 bis 0,01 Gew.-% (4,7-Dimethoxy-5-(prop-2-enyl)-2H(1,3-benzodioxol) und 0,001 bis 0,4 Gew.-%, vorzugsweise 0,005 bis 0,3 Gew.-%, besonders bevorzugt 0,01 bis 0,25 Gew.-%, weiter bevorzugt 0,05 bis 0,15 Gew.-% und insbesondere 0,05 bis 0,1 Gew.-% 2-methoxy-4-(prop-2-enyl)-phenol und 0,005 bis 0,3 Gew.-%, besonders bevorzugt 0,01 bis 0,25 Gew.-%, weiter bevorzugt 0,05 bis 0,15 Gew.-% und insbesondere 0,05 bis 0,1 Gew.-% 2-methoxy-4-(prop-1-enyl)-phenol oder
- 0,00002 bis 0,5 Gew.-%, vorzugsweise 0,00004 bis 0,25 Gew.-%, besonders bevorzugt 0,0001 bis 0,1 Gew.-%, weiter bevorzugt 0,00025 bis 0,05 Gew.-% und insbesondere 0,001 bis 0,01 Gew.-% (4,5-Dimethoxy-6-(prop-2-enyl)-2H(1,3-benzodioxol) und 0,001 bis 0,4 Gew.-%, vorzugsweise 0,005 bis 0,3 Gew.-%, besonders bevorzugt 0,01 bis 0,25 Gew.-%, weiter bevorzugt 0,05 bis 0,15 Gew.-% und insbesondere 0,05 bis 0,1 Gew.-% 2-methoxy-4-(prop-2-enyl)-phenol und 0,005 bis 0,3 Gew.-%, besonders bevorzugt 0,01 bis 0,25 Gew.-%, weiter bevorzugt 0,05 bis 0,15 Gew.-% und insbesondere 0,05 bis 0,1 Gew.-% 2-methoxy-4-(prop-1-enyl)-phenol oder
- 0,00002 bis 0,5 Gew.-%, vorzugsweise 0,00004 bis 0,25 Gew.-%, besonders bevorzugt 0,0001 bis 0,1 Gew.-%, weiter bevorzugt 0,00025 bis 0,05 Gew.-% und insbesondere 0,001 bis 0,01 Gew.-% (4,7-Dimethoxy-5-(prop-2-enyl)-2H(1,3-benzodioxol) und 0,00002 bis 0,5 Gew.-%, vorzugsweise 0,00004 bis 0,25 Gew.-%, besonders bevorzugt 0,0001 bis 0,1 Gew.-%, weiter bevorzugt 0,00025 bis 0,05 Gew.-% und insbesondere 0,001 bis 0,01 Gew.-% (4,5-Dimethoxy-6-(prop-2-enyl)-2H(1,3-benzodioxol) und 0,001 bis 0,4 Gew.-%, vorzugsweise 0,005 bis 0,3 Gew.-%, besonders bevorzugt 0,01 bis 0,25 Gew.-%, weiter bevorzugt 0,05 bis 0,15 Gew.-% und insbesondere 0,05 bis 0,1 Gew.-% 2-methoxy-4-(prop-2-enyl)-phenol und 0,005 bis 0,3 Gew.-%, besonders bevorzugt 0,01 bis 0,25 Gew.-%, weiter bevorzugt 0,05 bis 0,15 Gew.-% und insbesondere 0,05 bis 0,1 Gew.-% 2-methoxy-4-(prop-1-enyl)-phenol.

Erfindungsgemäße Mittel, die 0,00002 bis 0,5 Gew.-%, vorzugsweise 0,00004 bis 0,25 Gew.-%, besonders bevorzugt 0,0001 bis 0,1 Gew.-%, weiter bevorzugt 0,00025 bis 0,05 Gew.-% und insbesondere 0,001 bis 0,01 Gew.-% (4,7-Dimethoxy-5-(prop-2-enyl)-2H(1,3-benzodioxol) und 0,001 bis 0,4 Gew.-%, vorzugsweise 0,005 bis 0,3 Gew.-%, besonders bevorzugt 0,01 bis 0,25 Gew.-%, weiter bevorzugt 0,05 bis 0,15 Gew.-% und insbesondere 0,05 bis 0,1 Gew.-% 2-methoxy-4-(prop-2-enyl)-phenol enthalten, haben sich dabei als besonders wirksam gegen Halitosis erwiesen. Sie verringern nicht nur deutlich spürbar schlechten Atem nach der Anwendung, sondern besitzen auch einen langanhaltenden Effekt, der schlechten Atem nach der Nachtruhe ("bad morning breath") wirkungsvoll verhindert.

Unabhängig davon, welche Kombination der erfindungsgemäß notwendigen Inhaltsstoffe eingesetzt wird, ist es bevorzugt, die Verbindung(en) der der Gruppe b) mindestens in gleicher Menge, vorzugsweise im Überschuss zu der bzw. den Verbindung(en) aus der Gruppe a) einzusetzen. Hier haben sich erfindungsgemäße Mund- und Zahnpflege- und -reinigungsmittel als besonders bevorzugt erwiesen, bei denen das Gewichtssverhältnis von Verbindung(en) b) zu Verbindung(en) a) bei ≥ 1:1, vorzugsweise bei ≥ 1,2:1, besonders bevorzugt bei ≥ 1,4:1 und insbesondere bei ≥ 1,5:1 liegt.

Es hat sich gezeigt, dass die Wirkung der erfindungsgemäßen Kombination gegen Halitosis und insbesondere gegen Gingivitis und Parodontitis durch Zinksalze gesteigert werden kann.

Erfindungsgemäß bevorzugte Mittel enthalten daher mindestens ein Zinksalz, vorzugsweise in Mengen von 0,001 bis 5 Gew.-%, bezogen auf das gesamte Mittel wobei besonders bevorzugte Mund- und Zahnpflege- und -reinigungsmittel dadurch gekennzeichnet sind, dass sie 0,001 bis 5 Gew.-%, vorzugsweise 0,02 bis 2,5 Gew.-%, besonders bevorzugt 0,03 bis 2,0 Gew.-%, weiter bevorzugt 0,04 bis 1,5 Gew.-%, noch weiter bevorzugt 0,05 bis 1,0 Gew.-%, noch weiter bevorzugt 0,06 bis 0,5 Gew.-% und insbesondere 0,07 bis 0,25 Gew.-% mindestens eines Zinksalzes enthalten.

Hierbei gilt - wie immer, wenn in der vorliegenden Schrift keine anderen Angaben gemacht werden - dass sich die Mengenangaben auf das gesamte Mittel, d.h. das fertige Mund und Zahnpflege- und - reinigungsmittel, beziehen.

Erfindungsgemäß können sowohl anorganische als auch organische Salze des Zinks eingesetzt werden. Neben den nicht löslichen anorganischen Zinksalzen, also Salzen, welche eine Löslichkeit unterhalb 100 mg/L (20°C), vorzugsweise unterhalb 10 mg/L (20°C), insbesondere keine Löslichkeit (20°C) aufweisen (Bsp.: Zinkoxid), sind im Rahmen der vorliegenden Anmeldung die löslichen anorganischen Zinksalze, das heißt Salze, die in Wasser eine Löslichkeit oberhalb 100 mg/L, vorzugsweise oberhalb 500 mg/L, besonders bevorzugt oberhalb 1 g/L und insbesondere oberhalb 5 g/L aufweisen, bevorzugter Bestandteil erfindungsgemäßer Mittel. Zu den bevorzugten löslichen anorganischen Salzen zählen das Zinkbromid, das Zinkchlorid, das Zinkiodid, das Zinknitrat und das Zinksulfat.

Erfindungsgemäß besonders bevorzugte Mittel enthalten dabei mindestens ein Zinksalz aus der nachstehenden Tabelle:

| Zinksalz | Löslichkeit |
|---|---|
| Zinkacetat-Dihydrat | 430 g/l (20°C) |
| Zinkacetylacetonat | 4 g/l (20°C) |
| Zinkbromid | 820 g/l (25°C) |
| Zinkchlorid | 4320 g/l (25°C) |
| Zinkgluconat | 100 g/l (20°C) |
| Zinkhydroxycarbonat | Fast unlöslich (20°C) |
| Zinkiodid | 4500 g/l (20°C) |
| Zinknitrat Hexahydrat | 1843 g/l (20°C) |
| Zinknitrat-Tetrahydrat | Leicht löslich (20°C) |
| Zinkoxid | Unlöslich |
| Zinkstearat | 0,9 mg/l (20°C) |
| Zinksulfat-Heptahydat | 960 g/l (20°C) |
| Zinksulfat-Monohydrat | -350 g/l (20°C) |

Das Spektrum der erfindungsgemäß bevorzugten Zinksalze organischer Säuren, vorzugsweise organischer Carbonsäuren, reicht von Salzen die in Wasser nicht löslich sind, also eine Löslichkeit unterhalb 100 mg/L, vorzugsweise unterhalb 10 mg/L, insbesondere keine Löslichkeit aufweisen, bis zu solchen Salzen, die in Wasser eine Löslichkeit oberhalb 100 mg/L, vorzugsweise oberhalb 500 mg/L, besonders bevorzugt oberhalb 1 g/L und insbesondere oberhalb 5 g/L aufweisen (alle Löslichkeiten bei 20°C Wassertemperatur). Zu der ersten Gruppe von Zinksalzen gehören beispielsweise das Zinkcitrat, das Zinlaureat, das Zinkoleat, das Zinkoxalat, das Zinktartrat und das Zinkstearat, zu der Gruppe der löslichen organischen Zinksalze gehören beispielsweise das Zinkacetat, das Zinkacetylacetonat, das Zinkbenzoat, das Zinkformiat, das Zinklactat, das Zinkgluconat, das Zinkvalerat sowie das Zinksalz der p-Toluolsulfonsäure.

Es hat sich gezeigt, dass Zinksulfat ein besonders bevorzugt einzusetzendes Zinksalz ist. Zinksulfat bildet mehrere Hydrate. Das Heptahydrat bildet sich aus gesättigter wassriger Lösung in Form farbloser, glasglänzender, säulenförmiger, rhombischer Kristalle. Oberhalb 39°C erfolgt Umwandlung zu ZnSO₄ · 6H₂O, und bei 70°C liegt nur noch ZnSO₄ · H₂O vor; das letzte H₂O-Molekül entweicht bei 240°C. Interessanterweise steigt die erfindungsgemäße Wirkung bei den Zinksulfaten mit deren Kristallwassergehalt, so dass das Heptahydrat eine besonders bevorzugte Verbindung ist. Erfindungsgemäß bevorzugte Mund- und Zahnpflege- und -reinigungsmittel enthalten daher Zinksulfat, vorzugsweise Zinksulfat-Heptahydrat,

Unabhängig davon, welche(s) Zinksalz(e) und welche Kombination der erfindungsgemäß notwendigen Inhaltsstoffe eingesetzt wird, ist es bevorzugt, die Zinksalze mindestens in gleicher Menge, vorzugsweise im Überschuss zu den Verbindungen den Gruppen a) und b) einzusetzen. Hier haben sich erfindungsgemäße Mund- und Zahnpflege- und -reinigungsmittel als bevorzugt erwiesen, bei denen das Gewichtssverhältnis von Zinksalz(en) zu Verbindung(en) a) und b) bei ≥ 1:1, vorzugsweise bei ≥ 1,2:1, besonders bevorzugt bei ≥ 1,4:1 und insbesondere bei ≥ 1,5:1 liegt.

"Verbindung(en) a) und b)" bedeutet dabei in diesem Zusammenhang die Gesamtmenge an Verbindungen aus der Gruppe
- 4,7-Dimethoxy-5-(prop-2-enyl)-2H(1,3-benzodioxol)
- 4,5-Dimethoxy-6-(prop-2-enyl)-2H(1,3-benzodioxol)
- 2-methoxy-4-(prop-2-enyl)-phenol
- 2-methoxy-4-(prop-1-enyl)-phenol

Die Wirkung gegen Gingivitis und Parodontitis kann weiter gesteigert werden, wenn die erfindungsgemäßen Mittel mindestens ein Alkypyridiniumchlorid und/oder -bromid enthalten. Alkylpyridiniumhalogenide sind N-alkylsubstituierte Pyridine mit Halogenidionen als Gegenion. Bevorzugt sind dabei die Chloride.

Als Alkylreste kommen insbesondere C₈-C₂₄-Alkylreste in Frage, wobei lineare C₈-C₂₄-Alkylreste besonders bevorzugt sind. Ganz besonders bevorzugte Reste sind lineare C₁₀-Alkylreste, lineare C₁₂-Alkylreste, lineare C₁₄-Alkylreste, lineare C₁₆-Alkylreste, lineare C₁₈-Alkylreste und Mischungen der entsprechenden Alkylpyridiniumverbindungen, wobei ganz besonders bevorzugte erfindungsgemäße Mund- und Zahnpflege- und -reinigungsmittel - bezogen auf ihr Gewicht - 0,001 - 5 Gew.-%, vorzugsweise 0,005 - 2,5 Gew.-%, besonders bevorzugt 0,01 - 1 Gew.-%, außerordentlich bevorzugt 0,025 - 0,8 Gew.-% und insbesondere 0,05 bis 0,8 Gew.-% Cetylpyridiniumchlorid und/oder Laurylpyridiniumchlorid und/oder 1-Hexadecylpyridiniumchlorid oder deren Mischungen, enthalten.

Die erfindungsgemäße Kombination kann ihre vorteilhaften Wirkungen auf das Zahnfleisch und die Mundschleimhaut durch den Einsatz von Vitamin E noch weiter steigern.

Als weiteren Inhaltsstoff enthalten bevorzugte erfindungsgemäße Mund- und Zahnpflege- und -reinigungsmittel daher gegebenenfalls acetyliertes Vitamin E. Im Zuge der zu dieser Erfindung führenden Arbeiten hat sich herausgestellt, dass sich das bzw. die erfindungsgemäßen Mittel insbesondere dann besonders vitalisierend auf Zahnfleisch und Mundschleimhaut auswirken, wenn das gegebenenfalls acetylierte Vitamin E in bestimmten Konzentrationsbereichen eingesetzt wird. Mund- und Zahnpflege- und -reinigungsmittel, die, bezogen auf ihr Gesamtgewicht, 0,01 bis 1,0 Gew.-%, vorzugsweise 0,05 bis 0,5 Gew.-% gegebenenfalls acetyliertes Vitamin E enthalten, sind aus diesem Grund erfindungsgemäß bevorzugt.

Die Bezeichnung "gegebenenfalls acetyliertes Vitamin E" umfasst eine Reihe wirksamer Substanzen. Zu diesen Substanzen zählen insbesondere die Substanzfamilien der Tocopherole, der Tocotrienole sowie die Tocomonoenole und marinen Tocopherole (MDT, marine derived tocopherols).

Allen Vitamin-E-Formen gemeinsam ist ein an Position 6 hydroxyliertes Chromanring-Grundgerüst.

Die vier oben genannten Substanzfamilien unterscheiden sich hinsichtlich unterschiedlich gesättigter Seitenketten, wobei
- die Tocopherole eine gesättigte Seitenkette aufweisen;
- die Tocomonoenole und marinen Tocopherole eine einfach ungesättigte Seitenketten aufweisen; und
- die Tocotrienole eine dreifach gesättigte Seitenkette aufweisen.

In Abhängigkeit von der Methylierung des Chroman-Grundgerüsts wird in jeder der Substanzfamilien zwischen α-, β-, χ- und δ-Formen unterschieden.

So umfasst die Substanzfamilie der Tocopherole α-Tocopherol, β-Tocopherol, χ-Tocopherol, δ-Tocopherol sowie die weiteren natürlich vorkommenden Tocopherole 5,7-Dimethyltocol und 7-Methyltocol. Zur Substanzfamilie der Tocomonoenole zählt das α-Tocomonoenol, das β-Tocomonoenol, das χ-Tocomonoenol und das δ-Tocomonoenol. Die Substanzfamilie der marinen Tocopherole schließt α-MDT, β- MDT, χ- MDT und δ-MDT ein und die Substanzfamilie der Tocotrienole umfasst neben α-Tocotrienol, β-Tocotrienol, χ-Tocotrienol auch δ-Tocotrienol.

Alle vorgenannten Vitamin-E-Formen verbessern die Wirkung der erfindungsgemäßen Mittel auf Zahnfleisch und Mundschleimhaut. Aufgrund ihrer Wirkung besonders bevorzugt werden jedoch die Tocopherole, insbesondere des α-Tocopherol. Bevorzugte Mund- und Zahnpflege- und - reinigungsmittel sind dadurch gekennzeichnet, dass sie, bezogen auf ihr Gesamtgewicht, 0,001 bis 1,5 Gew.-%, vorzugsweise 0,01 bis 1,0 Gew.-%, und insbesondere 0,05 bis 0,5 Gew.-% gegebenenfalls acetyliertes α-Tocopherol enthalten.

Die Tocopherole weisen drei Stereozentren auf. Folglich existieren also von jedem der vier oben genannten Tocopherole (α-, β-, χ- und δ-Tocopherol) jeweils acht Stereoisomere. Als im Rahmen der vorliegenden Anmeldung besonders wirkungsvoll hat sich das RRR-α-Tocopherol erwiesen. Besonders bevorzugte Mund- und Zahnpflege- und -reinigungsmittel sind daher dadurch gekennzeichnet, dass sie, bezogen auf ihr Gesamtgewicht, 0,001 bis 1,5 Gew.-%, vorzugsweise 0,01 bis 1,0 Gew.-%, und insbesondere 0,05 bis 0,5 Gew.-% gegebenenfalls acetyliertes *RRR-*α-Tocopherol enthalten.

Da Vitamin E einschließlich der Tocopherole und des α-Tocopherols eine vergleichsweise geringe Stabilität aufweisen, wird im Rahmen der vorliegenden Anmeldung der Einsatz von acetyliertem Tocopherol (Tocopherylacetat), beispielsweise acetyliertem α-Tocopherol, insbesondere acetyliertem RRR-α-Tocopherol bevorzugt. Bevorzugte Mund- und Zahnpflege- und -reinigungsmittel sind dadurch gekennzeichnet, dass sie, bezogen auf ihr Gesamtgewicht, 0,001 bis 1,5 Gew.-%, vorzugsweise 0,01 bis 1,0 Gew.-%, und insbesondere 0,05 bis 0,5 Gew.-% Tocopherylacetat, vorzugsweise α-Tocopherylacetat, insbesondere RRR-α-Tocopherylacetat enthalten.

Die erfindungsgemäßen Mund- und Zahnpflege- und -reinigungsmittel können weitere Inhaltsstoffe enthalten. Bevorzugt ist hierbei der Einsatz von sogenannten Feuchthaltemitteln, die bei Zahnpasten das Austrocknen verhindern. Bei sogenannten Flüssigzahncremes mit fließfähiger Rheologie dienen diese als Matrix und werden in höheren Mengen eingesetzt. Bei Mundwässern und Mundspülungen dienen diese Alkohole als Konsistenzregler und zusätzliche Süßungsmittel.

Die erfindungsgemäßen Mund- und Zahnpflege- und -reinigungsmittel enthalten die - bezogen auf ihr Gewicht- 10 bis 50 Gew mehrwertige(n) Alkohol(e) aus der Gruppe Sorbit und/oder Glycerin und/oder 1,2-Propylenglycol oder deren Mischungen.

Hier sind erfindungsgemäße Mund- und Zahnpflege- und -reinigungsmittel bevorzugt, die - bezogen auf ihr Gewicht - 12,5 bis 45 Gew.-%, besonders bevorzugt 15 bis 40 Gew.-%, weiter bevorzugt 17,5 bis 35 Gew.-% und insbesondere 20 bis 29 Gew.-% mehrwertige(n) Alkohol(e) aus der Gruppe Sorbit und/oder Glycerin und/oder 1,2-Propylenglycol oder deren Mischungen enthalten.

Für bestimmte Anwendungsbereiche kann es vorteilhaft sein, nur einen der drei oben genannten Inhaltsstoffe einzusetzen. In den meisten Fällen ist dabei Sorbit bevorzugt. Allerdings können auf anderen Anwendungsgebieten Mischungen von zwei der drei Stoffe oder aller drei Stoffe bevorzugt sein. Besonders vorteilhaft hat sich hier eine Mischung aus Glycerin, Sorbit und 1,2-Propylenglycol in einem Gewichtsverhältnis von 1 : (0,5-1): (0,1-0,5) erwiesen.

Neben Sorbit bzw. Glycerin bzw. 1,2-Propylenglycol eignen sich als weitere mehrwertige Alkohole solche mit mindestens 2 OH-Gruppen, vorzugsweise Mannit, Xylitol, Polyethylenglycol, Polypropylenglycol und deren Mischungen. Unter diesen Verbindungen sind diejenigen mit 2 bis 12 OH-Gruppen und insbesondere diejenigen mit 2, 3, 4, 5, 6 oder 10 OH-Gruppen bevorzugt. Polyhydroxyverbindungen mit 2 OH-Gruppen sind beispielsweise Glycol (CH₂(OH)CH₂OH) und andere 1,2-Diole wie H-(CH₂)ₙ-CH(OH)CH₂OH mit n = 2, 3, 4, 5, 6, 7, 8, 9, 10, 11, 12, 13, 14, 15, 16, 17, 18, 19, 20. Auch 1,3-Diole wie H-(CH₂)ₙ-CH(OH) CH₂CH₂OH mit n = 1, 2, 3, 4, 5, 6, 7, 8, 9, 10, 11, 12, 13, 14, 15, 16, 17, 18, 19, 20 sind erfindungsgemäß einsetzbar. Die (n,n+1)- bzw. (n,n+2)-Diole mit nicht endständigen OH-Gruppen können ebenfalls eingesetzt werden. Wichtige Vertreter von Polyhydroxyverbindungen mit 2 OH-Gruppen sind auch die Polyethylen- und Polypropylenglycole. Als bevorzugte weitere mehrwertige Alkohole können z. B. Xylit, Propylenglycole, Polyethylenglycole, insbesondere solche mit mittleren Molekulargewichten von 200-800 eingesetzt werden.

Besonders bevorzugt ist der Einsatz von Sorbit, so dass Mittel, die außer Sorbit keine anderen mehrwertigen Alkohole enthalten, besonders bevorzugt sind.

Die erfindungsgemäßen Zusammensetzungen können zusätzlich mindestens ein Poliermittel enthalten. Als Poliermittel eignen sich prinzipiell alle für Zahnpasten bekannten Reibkörper, insbesondere solche, die keine Calciumionen enthalten. Bevorzugt geeignete Poliermittel-komponenten sind daher Kieselsäuren, Aluminiumhydroxid, Aluminiumoxid, Natrium-aluminiumsilikate, organische Polymere oder Gemische solcher Reibkörper.

Calciumhaltige Polierkomponenten wie z.B. Kreide, Calciumpyrophosphat, Dicalcium-phosphatdihydrat können aber in Mengen bis zu 5 Gew.-% - bezogen auf die Gesamtzusammensetzung - enthalten sein.

Der Gesamtgehalt an Poliermitteln liegt vorzugsweise im Bereich von 5 - 50 Gew.-% des Zahnpflegemittels. Erfindungsgemäß bevorzugte Mittel enthalten Poliermittel innerhalb engerer Mengenbereiche. Hier sind erfindungsgemäß bevorzugte Mund- und Zahnpflege- und - reinigungsmittel dadurch gekennzeichnet, dass sie - bezogen auf ihr Gewicht - 1 bis 25 Gew.-%, vorzugsweise 2,5 bis 20 Gew.-%, weiter bevorzugt 5 bis 18 Gew.-% und insbesondere 7,5 bis 16 Gew.-% Poliermittel enthalten.

Besonders geeignet sind Zahnpasten und flüssige Zahnreinigungsmittel, die als Poliermittel Kieselsäuren enthalten. Geeignete Kieselsäuren sind z.B. Gelkieselsäuren, Hydrogelkieselsäuren und Fällungskieselsäuren. Gelkieselsäuren werden durch Umsetzung von Natriumsilikatlösungen mit starken, wassrigen Mineralsäuren unter Ausbildung eines Hydrosols, Alterung zum Hydrogel, Waschen und Trocknen hergestellt. Erfolgt die Trocknung unter schonenden Bedingungen auf Wassergehalte von 15 bis 35 Gew.-%, so werden die so genannten Hydrogelkieselsäuren erhalten. Durch Trocknung auf Wassergehalte unterhalb 15 Gew.-% erfolgt eine irreversible Schrumpfung der vorher lockeren Struktur des Hydrogels zur dichten Struktur des sog. Xerogels.

Eine zweite, besonders geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Bevorzugt geeignet ist eine Fällungskieselsäure mit einer BET-Oberfläche von 15 - 110 m²/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20°C) in einer Menge von 10 - 20 Gew.-% der Zahnpaste. Bevorzugt geeignete Fällungskieselsäuren dieser Art weisen außerdem gerundete Ecken und Kanten auf und sind unter der Handelsbezeichnung Sident® 12 DS (DEGUSSA) erhältlich.

Andere Fällungskieselsäuren dieser Art sind Sident® 8 (Evonik) und Sorbosil® AC 39 (PQ Corporation). Diese Kieselsäuren zeichnen sich durch eine geringere Verdickungswirkung und eine etwas höhere mittlere Teilchengröße von 8 --14 µm bei einer spezifischen Oberfläche von 40 - 75 m²/g (nach BET) aus und eignen sich besonders gut für flüssige Zahncremes. Diese sollten eine Viskosität (25°C, Scherrate D = 10 s⁻¹) von 10 - 100 Pas aufweisen.

Zahnpasten, die eine deutlich höhere Viskosität von mehr als 100 Pas (25° C, D = 10 s⁻¹) aufweisen, benötigen hingegen einen genügend hohen Anteil an Kieselsäuren mit einer Teilchengröße von weniger als 5 µm, bevorzugt wenigstens 3 Gew.-% einer Kieselsäure mit einer Partikelgröße von 1 - 3 µm. Solchen Zahnpasten setzt man daher bevorzugt neben den genannten Fällungskieselsäuren noch feinteiligere, so genannte Verdickungs-Kieselsäuren mit einer BET-Oberfläche von 150 - 250 m²/g zu, z.B. das Handelsprodukt Sident® 22S.

Als weitere Poliermittelkomponente kann auch z.B. Aluminiumoxid in Form von schwach calcinierter Tonerde mit einem Gehalt an - und -Aluminiumoxid in einer Menge von ca. 1 - 5 Gew.-% enthalten sein. Ein solches geeignetes Aluminiumoxid ist unter der Handelsbezeichnung "Poliertonerde P10 feinst" (Giulini Chemie) erhältlich.

Als Poliermittel eignen sich weiter alle für Zahnpasten bekannten Reibkörper wie z. B. Natriumaluminiumsilikate wie z. B. Zeolith A, organische Polymere wie z. B. Polymethacrylat oder Gemische dieser und der vorstehend genannten Reibkörper.

Zusammenfassend sind erfindungsgemäße Mund- und Zahnpflege- und -reinigungsmittel bevorzugt, die zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ ·2H₂O), Natriumaluminiumsilikate, insbesondere Zeolith A, organische Polymere, insbesondere Polymethacrylate oder Gemische dieser Reibkörper, vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthalten.

Erfindungsgemäß besonders bevorzugte Mund und Zahnpflege- und -reinigungsmittel enthalten ausschließlich Poliermittel aus der Gruppe der Kieselsäuren, Aluminiumhydroxid, Aluminiumoxid, Dicalciumphosphat-dihydrat (CaHPO₄ ·2H₂O) oder Gemische dieser Reibkörper. Diese Poliermittel haben sich als besonders effizient bei der Lösung der erfindungsgemäßen Aufgabe erwiesen.

Ganz besonders bevorzugt enthalten die erfindungsgemäßen Zusammensetzungen bezogen auf ihr Gewicht 1 bis 30 Gew.-% Fällungskieselsäure(n) mit einer spezifischen Oberfläche nach ISO 5794-1, Anhang D von ≤ 55 m²/g. Vorzugsweise werden die Fällungskieselsäuren, die entsprechende spezifische Oberflächen aufweisen, innerhalb engerer Mengenbereiche eingesetzt, und insbesondere bevorzugt werden Fällungskieselsäuren eingesetzt, die noch niedrigere spezifische Oberflächen nach ISO 5794-1, Anhang D aufweisen. Bevorzugte erfindungsgemäße Mund- und Zahnpflege- und - reinigungsmittel enthalten 2,5 bis 25 Gew.-%, vorzugsweise 5 bis 20 Gew.-%, besonders bevorzugt 7,5 bis 17,5 Gew.-%, weiter bevorzugt 8,0 bis 15,0 Gew.-% und insbesondere 10,0 bis 13,0 Gew.-% Fällungskieselsäure(n) mit einer spezifischen Oberfläche nach ISO 5794-1, Anhang D von ≤ 55 m²/g. Besonders bevorzugte erfindungsgemäße Mund und Zahnpflege- und -reinigungsmittel sind dadurch gekennzeichnet, dass sämtliche im Mittel enthaltenen Fällungskieselsäure(n) eine spezifische Oberfläche nach ISO 5794-1, Anhang D von ≤ 53 m²/g, vorzugsweise von ≤ 51 m²/g, weiter bevorzugt von ≤ 49 m²/g und insbesondere von ≤ 47 m²/g aufweisen.

Als weiteren wesentlichen Inhaltsstoff können die erfindungsgemäßen Mund- und Zahnpflege- und - reinigungsmittel mindestens ein anionisches Tensid enthalten.

Geeignete anionische Tenside sind z. B. lineare Natriumalkylsulfate mit 12-18 C-Atomen in der Alkylgruppe. Diese Stoffe weisen zusätzlich eine enzymhemmende Wirkung auf den bakteriellen Stoffwechsel des Zahnbelags auf. Weitere geeignete Tenside sind Alkalisalze, bevorzugt Natriumsalze von Alkylpolyglycolethersulfat mit 12-16 C-Atomen in der linearen Alkylgruppe und 2-6 Glycolethergruppen im Molekül, von linearem Alkan(C₁₂-C₁₈)-sulfonat, von Sulfobernsteinsäure-monoalkyl(C₁₂-C₁₈)-estern, von sulfatisierten Fettsäuremonoglyceriden, sulfatisierten Fettsäurealkanolamiden, Sulfoessigsäurealkyl(C₁₂-C₁₆)-estern, Acylsarcosinen, Acyltauriden und Acylisothionaten mit jeweils 8-18 C-Atomen in der Acylgruppe.

Weitere typische Beispiele für anionische Tenside sind Seifen, Alkylbenzolsulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkyl-sulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Erfindungsgemäß bevorzugte Mund- und Zahnpflege- und -reinigungsmittel sind dadurch gekennzeichnet, dass sie, bezogen auf ihr Gesamtgewicht, 0,05 bis 5,0 Gew.-%, vorzugsweise 0,1 bis 3,5 Gew.-%, besonders bevorzugt 0,2 bis 2,5 Gew.-% und insbesondere 0,5 bis 2,0 Gew.-% anionische(s) Tensid(e), vorzugsweise Natriumlaurylsulfat enthalten.

Ein besonders vorteilhafter Effekt auf Zahnfleisch und Mundschleimhaut ist dann zu beobachten, wenn die Mittel sowohl anionische(s) Tensid(e) und Vitamin E enthalten und die Gesamtmenge an anionischen Tensiden, die in den erfindungsgemäßen Mitteln eingesetzt wird, auf die im Mittel enthaltende Menge an Vitamin E (a) abgestimmt ist. Vorzugsweise beträgt die Menge an Aniontensiden dabei maximal das 20-fache der Menge an Vitamin E, weiter bevorzugt maximal das 18-fache, noch weiter bevorzugt maximal das 16-fache und insbesondere maximal das 15-fache der menge an Vitamin E.

Als weiteren Inhaltsstoff können die erfindungsgemäßen Mund- und Zahnpflege- und - reinigungsmittel mindestens ein amphoteres Tensid enthalten.

Amphotere Tenside werden in ampholytische Tenside und zwitterionische Tenside unterteilt. Unter ampholytischen Tensiden werden solche oberflächenaktiven Verbindungen verstanden, die sowohl saure als auch basische hydrophile Gruppen besitzen und sich also je nach Bedingung sauer oder basisch verhalten.

Geeignete ampholytische Tenside enthalten beispielsweise außer einer C₈ - C₂₄ - Alkyl- oder -Acylgruppe mindestens eine freie Aminogruppe (basische hydrophile Gruppe) und mindestens eine - COOH- oder -SO₃H-Gruppe (saure hydrophile Gruppe) und sind zur Ausbildung innerer Salze befähigt. Beispiele für geeignete ampholytische Tenside sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.

Besonders bevorzugte erfindungsgemäße Mund und Zahnpflege- und -reinigungsmittel sind dadurch gekennzeichnet, dass sie amphotere(s) Tensid(e) aus den Gruppen der
- N-Alkylglycine,
- N-Alkylpropionsäuren,
- N-Alkylaminobuttersäuren,
- N-Alkyliminodipropionsäuren,
- N-Hydroxyethyl-N-alkylamidopropylglycine,
- N-Alkyltaurine,
- N-Alkylsarcosine,
- 2-Alkylaminopropionsäuren mit jeweils etwa 8 bis 24 C-Atomen in der Alkylgruppe,
- Alkylaminoessigsäuren mit jeweils etwa 8 bis 24 C-Atomen in der Alkylgruppe,
- N-Kokosalkylaminopropionat,
- Kokosacylaminoethylaminopropionat
- C₁₂ - C₁₈ -Acylsarcosin,
- N-Alkyl-N,N-dimethylammonium-glycinate, beispielsweise Kokosalkyl-dimethylammoniumglycinat,
- N-Acyl-aminopropyl-N,N-dimethylammoniumglycinate, beispielsweise Kokosacylaminopropyldimethylammoniumglycinat,
- 2-Alkyl-3-carboxymethyl-3-hydroxyethyl-imidazoline mit jeweils 8 bis 18 C-Atomen in der Alkyl-oder Acylgruppe
- Kokosacylaminoethylhydroxyethylcarboxymethylglycinat
- der unter der INCI-Bezeichnung Cocamidopropyl Betain bekannten Verbindungen,
- der unter der INCI-Bezeichnung Disodium Cocoamphodiacetate bekannten Verbindungen enthalten.

Weiterhin ebenfalls bevorzugte Mund und Zahnpflege- und -reinigungsmittel enthalten als amphotere Tenside Betaine der Formel (Bet-I) 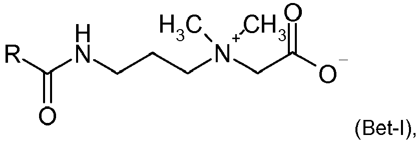 in der R für einen geradkettigen oder verzweigten, gesättigten oder ein- bzw. mehrfach ungesättigten Alkyl- oder Alkenylrest mit 8 bis 24 Kohlenstoffatomen steht.

Diese Tenside werden nach der INCI-Nomenklatur als Amidopropylbetaine bezeichnet, wobei die Vertreter, die sich von Kokosfettsäuren ableiten, bevorzugt sind und als Cocoamidopropylbetaine bezeichnet werden. Besonders bevorzugt werden erfindungsgemäß Tenside der Formel (Bet-I) eingesetzt, die ein Gemisch der folgenden Vertreter sind:
H₃C-(CH₂)₇-C(O)-NH-(CH₂)₃N⁺(CH₃)₂CH₂COO⁻
H₃C-(CH₂)₉-C(O)-NH-(CH₂)₃N⁺(CH₃)₂CH₂COO⁻
H₃C-(CH₂)₁₁-C(O)-NH-(CH₂)₃N⁺(CH₃)₂CH₂COO-
H₃C-(CH₂)₁₃-C(O)-NH-(CH₂)₃N⁺(CH₃)₂CH₂COO-
H₃C-(CH₂)₁₅-C(O)-NH-(CH²)₃N⁺(CH₃)₂CH₂COO-
H₃C-(CH₂)₇-CH=CH-(CH₂)₇-C(O)-NH-(CH₂)₃N⁺(CH₃)₂CH₂COO-

Wenn die Mitte sowohl Amphotenside als auch Vitamin E enthalten und die Gesamtmenge der im erfindungsgemäßen Mittel eingesetzten amphoteren Tenside auf die im Mittel enthaltene Menge an gegebenenfalls acetyliertem Vitamin E (a) abgestimmt ist, so ist dies ebenfalls von besonderem Vorteil im Hinblick auf die Vitalisierung von Zahnfleisch und Mundschleimhaut. Aus diesem Grund ist es bevorzugt, wenn die amphoteren Tenside - bezogen auf ihre Gesamtmenge im Mittel - in speziellen Mengenbereichen eingesetzt werden.

In einer weiteren besonders bevorzugten Ausführungsform ist ein erfindungsgemäßes Mund- und Zahnpflege- und -reinigungsmittel daher dadurch gekennzeichnet, dass es, bezogen auf sein Gesamtgewicht, 0,05 bis 5,0 Gew.-%, vorzugsweise 0,1 bis 3,5 Gew.-%, besonders bevorzugt 0,2 bis 2,5 Gew.-% und insbesondere 0,5 bis 2,0 Gew.-% amphotere(s) Tensid(e), vorzugsweise Cocamidopropylbetain enthält.

Die erfindungsgemäß bevorzugte Kombination aus anionischem und amphoterem Tensid ist schonend für das Zahnfleisch und stellt gleichzeitig eine ausreichende Reinigung der Zahnoberflächen sicher. Für die Wirkung der erfindungsgemäßen Mittel hat es sich als vorteilhaft erwiesen, die zuvor beschriebene Wirkstoffkombination aus Vitamin E, anionischem Tensid und amphoterem Tensid durch weitere spezifische Wirkstoffe zu ergänzen, wobei sich wundheilende und entzündungshemmende Stoffe und Fluoridverbindungen als besonderes wirkungsvoll erwiesen haben.

Als wundheilende und entzündungshemmende Stoffe eignen sich beispielsweise Allantoin, Azulen, Kamillenextrakten, Tocopherol, Panthenol, Bisabolol, insbesondere jedoch Salbeiextrakte. Mund- und Zahnpflege- und -reinigungsmittel nach einem der vorherigen Ansprüche, dadurch gekennzeichnet, dass sie, bezogen auf ihr Gesamtgewicht, 0,001 bis 2,0 Gew.-%, vorzugsweise 0,01 bis 1,0 Gew.-% und insbesondere 0,05 bis 0,25 Gew.-% Salbei-Blattextrakt enthalten, werden erfindungsgemäß bevorzugt.

Ein Extrakt im Sinne der vorliegenden Anmeldung ist ein Stoff oder Stoffgemisch, welches durch Extraktion gewonnen wurde. Die Angaben zum Gewichtsanteil des Extrakts am Gesamtgewicht erfindungsgemäßer Mittel beziehen sich also auf die aus dem Extraktionsgut durch Extraktion erhaltenen Stoffe oder Stoffgemische nicht jedoch auf etwaige Hilfs- oder Begleitstoffe, wie die zur Extraktion eingesetzten Lösungsmittel.

Man unterscheidet nach der Beschaffenheit *Trockenextrakte* d.h. bis zur Trockene eingedampfte Extrakte, *Fluidextrakte* d.h. mit Lösungsmitteln so hergestellte Extrakte, dass aus einem Teil Droge höchstens 2 Teile Fluid-Extrakt gewonnen werden, *zähflüssige Extrakte bzw. Dickextrakte,* d.h. Extrakte, bei denen ein Teil des Lösungsmittels verdampft wird.

Extrakte des Salbeis werden vor allem aus den Blättern gewonnen. Erfindungsgemäß geeignet sind sämtliche Extrakte, aus Kostengründen sind Extrakte aus den Blättern.

Die erfindungsgemäß verwendeten Extrakte werden durch Extraktion vorzugsweise mit organischen Lösemitteln, Wasser oder Gemischen daraus, gewonnen. Bevorzugt geeignete organische Lösemittel sind Ketone (z.B. Aceton), Ether, Ester, Alkohole oder halogenierte Kohlenwasserstoffe. Besonders bevorzugte Extraktionsmittel sind Wasser und/oder Alkohole. Unter den Alkoholen sind dabei (C₁ bis C₆)- Alkohole, wie Ethanol und Isopropanol und zwar sowohl als alleiniges Extraktionsmittel als auch in Mischung mit Wasser, bevorzugt. Auch solventfreie Extraktionsmethoden, einschließlich der überkritischen CO₂-Extraktion sind einsetzbar.

Mund- und Zahnpflege- und reinigungsmittel, insbesondere Zahnpasten, können zusätzlich auch Substanzen enthalten, die gegen Plaque und/oder Zahnstein wirksam sind. Hierzu eignen sich beispielsweise antimikrobielle Stoffe und Konsvervierungsmittel (Plaque) oder Chelatbildner (Zahnstein).

Erfindungsgemäß bevorzugte Mund- und Zahnpflege- und -reinigungsmittel sind dadurch gekennzeichnet, dass sie zusätzlich Antiplaque-Wirkstoffe, vorzugsweise Alkypyridiniumsalze, insbesondere Cetylpyridiniumchlorid, p-Hydroxybenzoesäuremethyl-, -ethyl- oder propylester, Natriumsorbat, Natriumbenzoat, Bromchlorophen, Triclosan, Hexetidine, Phenyl- Salicylsäureester, Biguanide z. B. Chlorhexidin, Thymol, vorzugsweise in Mengen von 0,05 bis 5 Gew.%, vorzugsweise von 0,10 bis 2,5 Gew.% und insbesondere von 0,30 bis 1,5 Gew.%, jeweils bezogen auf das gesamte Mittel, enthalten.

Gegen Zahnstein wirksame Stoffe können beispielsweise Chelatbildner sein wie z. B. Ethylendiamintetraessigsäure und deren Natriumsalze, Pyrophosphat- Salze wie die wasserlöslichen Dialkali- oder Tetraalkalimetallpyrophosphat- Salze, z. B. Na₄P₂O₇, K₄P₂O₇, Na₂K₂P₂O₇, Na₂H₂P₂O₇ und K ₂H₂P₂O₇ oder Polyphosphat-Salze, die z. B. aus wasserlöslichen Alkalimethalltripolyphosphaten wie Natriumtripolyphosphat und Kaliumtripolyphosphat ausgewählt sein können. Erfindungsgemäß bevorzugte Mund- und Zahnpflege- und -reinigungsmittel sind dadurch gekennzeichnet, dass sie zusätzlich Phosphat(e), vorzugsweise Alkalimetallphosphat(e) und insbesondere Natriumtripolyphosphat, vorzugsweise in Mengen von 1 bis 10 Gew.-%, besonders bevorzugt von 2 bis 8 Gew.-% und insbesondere von 3 bis 7 Gew.-%, jeweils bezogen auf das gesamte Mittel, enthalten. Die erfindungsgemäßen Zahnpflegemittel können z.B. durch Zusatz von Aromaölen und Süßungsmitteln in ihren organoleptischen Eigenschaften verbessert werden.

Als Aromaöle können alle die für Mund- und Zahnpflegemittel üblichen natürlichen und synthetischen Aromen eingesetzt werden. Natürliche Aromen können sowohl in Form der aus Drogen isolierten natürlichen ätherischen Öle als auch der daraus isolierten Einzelkomponenten enthalten sein. Geeignete Aromen sind z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser Komponenten. Geeignete Süßungsmittel sind z.B. Saccharin-Natrium, Natrium-Cyclamat, Sucrose, Lactose, Meltose, Fructose sowie Maltose und Dextrose.

Die erfindungsgemäßen Mittel können in einem kosmetischen, nicht therapeutischen Verfahren zur Reinigung der Zähne bei gleichzeitiger Vitalisierung bzw. Kräftigung des Zahnfleisches und der Mundschleimhaut eingesetzt werden

Erfindungsgemäße Mittel können als Zahnpasten oder Zahncremes formuliert werden. Ein weiterer Gegenstand der vorliegenden Erfindung ist die Verwendung von erfindungsgemäßen Mitteln zur Reinigung von Zähnen mittels manuell betätigter oder elektrischer Zahnbürsten. Im Falle elektrischer Zahnbürsten besitzen die erfindungsgemäßen Mittel den weiteren Vorteil, dass sie bereits in geringen Mengen wirksam sind und darüber hinaus die Mechanik des elektrischen Bürstenkopfes nicht beeinträchtigen.

Ein weiterer Gegenstand der vorliegenden Erfindung ist ein Verfahren zum Reinigen von Zähnen, dadurch gekennzeichnet, dass ein erfindungsgemäßes Mittel auf den Bürstenkopf einer elektrischen Zahnbürste aufgetragen und mit der elektrischen Zahnbürste die Zähne geputzt werden.

Ein weiterer Gegenstand der vorliegenden Erfindung ist ein kosmetisches, nicht-herapeutisches Verfahren zur Bekämpfung von Halitosis und/oder zur Behandlung von Gingivitis oder Parodontitis, bei dem eine erfindungsgemäße Zubereitung auf eine Zahnbürste aufgetragen und mit dieser die Zähne geputzt werden.

Bezüglich bevorzugter Ausführungsformen des erfindungsgemäßen Verfahrens gilt mutatis mutandis das zu den erfindungsgemäßen Mitteln Gesagte.

Durch den Einsatz der erfindungsgemäßen Kombination wird Mundgeruch auch über einen langen Zeitraum effektiv verringert bzw. verhindert. Darüber hinaus besitzt die erfindungsgemäße Kombination eine hohe Wirksamkeit gegebn Gingivitis und Parodontitis.

## Patentansprüche

1. Mund- und Zahnpflege- und -reinigungsmittel, enthaltend - bezogen auf sein Gewicht -
a) 0,00001 bis 1 Gew.-% Verbindung(en) aus der Gruppe
a. 4,7-Dimethoxy-5-(prop-2-enyl)-2H(1,3-benzodioxol) und/oder
b. 4,5-Dimethoxy-6-(prop-2-enyl)-2H(1,3-benzodioxol) und/oder
c. Mischungen aus diesen;
b) 0,001 bis 1 Gew.-% Verbindung(en) aus der Gruppe
d. 2-methoxy-4-(prop-2-enyl)-phenol und/oder
e. 2-methoxy-4-(prop-1-enyl)-phenol und/oder
f. Mischungen aus diesen,
c) 10 bis 50 Gew mehrwertige(n) Alkohol(e) aus der Gruppe Sorbit und/oder Glycerin und/oder 1,2-Propylenglycol oder deren Mischungen
mit der Maßgabe, daß das Mund- und Zahnpflege- und -reinigungsmittel 0,05 bis 0,4 Gew.-%, 2-methoxy-4-(prop-1-enyl)-phenol 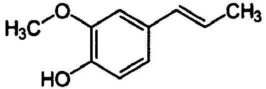 enthält.

2. Mund- und Zahnpflege- und -reinigungsmittel nach Anspruch 1, **dadurch gekennzeichnet, dass** es 0,000025 bis 0,5 Gew.-%, vorzugsweise 0,00005 bis 0,25 Gew.-%, besonders bevorzugt 0,0001 bis 0,1 Gew.-%, weiter bevorzugt 0,0005 bis 0,05 Gew.% und insbesondere 0,001 bis 0,01 Gew.% 4,7-Dimethoxy-5-(prop-2-enyl)-2H(1,3-benzodioxol) enthält.

3. Mund- und Zahnpflege- und -reinigungsmittel nach einem der Ansprüche 1 oder 2, **dadurch gekennzeichnet, dass** es 0,0025 bis 0,5 Gew.-%, vorzugsweise 0,05 bis 0,4 Gew.-%, besonders bevorzugt 0,01 bis 0,3 Gew.-%, weiter bevorzugt 0,05 bis 0,2 Gew.-% und insbesondere 0,075 bis 0,1 Gew.-% 2-methoxy-4-(prop-2-enyl)-phenol enthält.

4. Mund- und Zahnpflege- und -reinigungsmittel nach einem der Ansprüche 1 bis 3, **dadurch gekennzeichnet, dass** es 0,00004 bis 0,25 Gew.-%, besonders bevorzugt 0,0001 bis 0,1 Gew.-%, weiter bevorzugt 0,00025 bis 0,05 Gew.-% und insbesondere 0,001 bis 0,01 Gew.-% (4,7-Dimethoxy-5-(prop-2-enyl)-2H(1,3-benzodioxol) und 0,001 bis 0,4 Gew.-%, vorzugsweise 0,005 bis 0,3 Gew.-%, besonders bevorzugt 0,01 bis 0,25 Gew.%, weiter bevorzugt 0,05 bis 0,15 Gew.-% und insbesondere 0,05 bis 0,1 Gew.-% 2-methoxy-4-(prop-2-enyl)-phenol enthält.

5. Mund- und Zahnpflege- und -reinigungsmittel nach einem der Ansprüche 1 bis 4, **dadurch gekennzeichnet, dass** das Gewichtsverhältnis von Verbindung(en) b) zu Verbindung(en) a) bei ≥ 1:1, vorzugsweise bei ≥ 1,2:1, besonders bevorzugt bei ≥ 1,4:1 und insbesondere bei ≥ 1,5:1 liegt.

6. Mund- und Zahnpflege- und -reinigungsmittel nach einem der Ansprüche 1 bis 5, **dadurch gekennzeichnet, dass** es 0,001 bis 5 Gew.-%, vorzugsweise 0,02 bis 2,5 Gew.-%, besonders bevorzugt 0,03 bis 2,0 Gew.%, weiter bevorzugt 0,04 bis 1,5 Gew.%, noch weiter bevorzugt 0,05 bis 1,0 Gew.-%, noch weiter bevorzugt 0,06 bis 0,5 Gew.-% und insbesondere 0,07 bis 0,25 Gew.% mindestens eines Zinksalzes enthält.

7. Mund- und Zahnpflege- und -reinigungsmittel nach einem der Ansprüche 1 bis 6, **dadurch gekennzeichnet, dass** es Zinksulfat, vorzugsweise Zinksulfat-Heptahydrat, enthält.

8. Mund- und Zahnpflege- und -reinigungsmittel nach einem der Ansprüche 6 bis 7, **dadurch gekennzeichnet, dass** das Gewichtssverhältnis von Zinksalz(en) zu Verbindung(en) a) und b) bei ≥ 1:1, vorzugsweise bei ≥ 1,2:1, besonders bevorzugt bei ≥ 1,4:1 und insbesondere bei ≥ 1,5:1 liegt.

9. Mund- und Zahnpflege- und -reinigungsmittel nach einem der Ansprüche 1 bis 8, **dadurch gekennzeichnet, dass** es 0,001 - 5 Gew.%, vorzugsweise 0,005 - 2,5 Gew.%, besonders bevorzugt 0,01 - 1 Gew.-%, außerordentlich bevorzugt 0,025 - 0,8 Gew.% und insbesondere 0,05 bis 0,8 Gew.-% Cetylpyridiniumchlorid und/oder Laurylpyridiniumchlorid und/oder 1-Hexadecylpyridiniumchlorid oder deren Mischungen, enthält.

10. Mund- und Zahnpflege- und -reinigungsmittel nach einem der Ansprüche 1 bis 9, **dadurch gekennzeichnet, dass** es bezogen auf sein Gesamtgewicht, 0,05 bis 5,0 Gew.-%, vorzugsweise 0,1 bis 3,5 Gew.-%, besonders bevorzugt 0,2 bis 2,5 Gew.% und insbesondere 0,5 bis 2,0 Gew.-% amphotere(s) Tensid(e), vorzugsweise Cocamidopropylbetain enthält.

11. Mund- und Zahnpflege- und -reinigungsmittel nach einem der Ansprüche 1 bis 10, **dadurch gekennzeichnet, dass** sie, bezogen auf ihr Gesamtgewicht, 0,05 bis 5,0 Gew.-%, vorzugsweise 0,1 bis 3,5 Gew.-%, besonders bevorzugt 0,2 bis 2,5 Gew.% und insbesondere 0,5 bis 2,0 Gew.-% anionische(s) Tensid(e), vorzugsweise Natriumlaurylsulfat enthalten.

## Claims

1. An oral and dental care and cleaning agent, containing, based on its weight:
a) from 0.00001 to 1 wt.% compound(s) from the group comprising
a. 4,7-dimethoxy-5-(prop-2-enyl)-2H(1,3-benzodioxole) and/or
b. 4,5-dimethoxy-6-(prop-2-enyl)-2H(1,3-benzodioxole) and/or
c. mixtures thereof;
b) from 0.001 to 1 wt.% compound(s) from the group comprising
d. 2-methoxy-4-(prop-2-enyl)-phenol and/or
e. 2-methoxy-4-(prop-1-enyl)-phenol and/or
f. mixtures thereof,
c) from 10 to 50 wt.% polyvalent alcohol(s) from the group comprising sorbitol and/or glycerol and/or 1,2-propylene glycol or mixtures thereof,
with the proviso that the oral and dental care and cleaning agent contains from 0.05 to 0.4 wt.% 2-methoxy-4-(prop-1-enyl)-phenol.

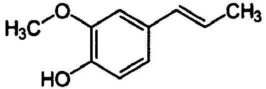

2. The oral and dental care and cleaning agent according to claim 1, **characterized in that** it contains from 0.000025 to 0.5 wt.%, preferably from 0.00005 to 0.25 wt.%, particularly preferably from 0.0001 to 0.1 wt.%, more preferably from 0.0005 to 0.05 wt.%, and in particular from 0.001 to 0.01 wt.%, 4,7-dimethoxy-5-(prop-2-enyl)-2H(1,3-benzodioxole).

3. The oral and dental care and cleaning agent according to one of claims 1 or 2, **characterized in that** it contains from 0.0025 to 0.5 wt.%, preferably from 0.05 to 0.4 wt.%, particularly preferably from 0.01 to 0.3 wt.%, more preferably from 0.05 to 0.2 wt.%, and in particular from 0.075 to 0.1 wt.%, 2-methoxy-4-(prop-2-enyl)-phenol.

4. The oral and dental care and cleaning agent according to one of claims 1 to 3, **characterized in that** it contains from 0.00004 to 0.25 wt.%, particularly preferably from 0.0001 to 0.1 wt.%, more preferably from 0.00025 to 0.05 wt.%, and in particular from 0.001 to 0.01 wt.%, 4,7-dimethoxy-5-(prop-2-enyl)-2H(1,3-benzodioxole) and from 0.001 to 0.4 wt.%, preferably from 0.005 to 0.3 wt.%, particularly preferably from 0.01 to 0.25 wt.%, more preferably from 0.05 to 0.15 wt.%, and in particular from 0.05 to 0.1 wt.%, 2-methoxy-4-(prop-2-enyl)-phenol.

5. The oral and dental care and cleaning agent according to one of claims 1 to 4, **characterized in that** the weight ratio of compound(s) b) to compound(s) a) is ≥ 1:1, preferably ≥ 1.2:1, particularly preferably ≥ 1.4:1 and in particular ≥ 1.5:1.

6. The oral and dental care and cleaning agent according to one of claims 1 to 5, **characterized in that** it contains from 0.001 to 5 wt.%, preferably from 0.02 to 2.5 wt.%, particularly preferably from 0.03 to 2.0 wt.%, more preferably from 0.04 to 1.5 wt.%, even more preferably from 0.05 to 1.0 wt.%, even more preferably from 0.06 to 0.5 wt.%, and in particular from 0.07 to 0.25 wt.%, of at least one zinc salt.

7. The oral and dental care and cleaning agent according to one of claims 1 to 6, **characterized in that** it contains zinc sulfate, preferably zinc sulfate heptahydrate.

8. The oral and dental care and cleaning agent according to one of claims 6 to 7, **characterized in that** the weight ratio of zinc salt(s) to compound(s) a) and b) is ≥ 1:1, preferably ≥ 1.2:1, particularly preferably ≥ 1.4:1 and in particular ≥ 1.5:1.

9. The oral and dental care and cleaning agent according to one of claims 1 to 8, **characterized in that** it contains from 0.001 to 5 wt.%, preferably from 0.005 to 2.5 wt.%, particularly preferably from 0.01 to 1 wt.%, extremely preferably from 0.025 to 0.8 wt.%, and in particular from 0.05 to 0.8 wt.%, cetylpyridinium chloride and/or laurylpyridinium chloride and/or 1-hexadecylpyridinium chloride or mixtures thereof.

10. The oral and dental care and cleaning agent according to one of claims 1 to 9, **characterized in that** it contains, based on its total weight, from 0.05 to 5.0 wt.%, preferably from 0.1 to 3.5 wt.%, particularly preferably from 0.2 to 2.5 wt.%, and in particular from 0.5 to 2.0 wt.%, amphoteric surfactant(s), preferably cocamidopropyl betaine.

11. The oral and dental care and cleaning agent according to one of claims 1 to 10, **characterized in that** it contains, based on its total weight, from 0.05 to 5.0 wt.%, preferably from 0.1 to 3.5 wt.%, particularly preferably from 0.2 to 2.5 wt.%, and in particular from 0.5 to 2.0 wt.%, anionic surfactant(s), preferably sodium lauryl sulfate.

## Revendications

1. Agent de soin et de nettoyage buccodentaire contenant, par rapport à son poids,
a) de 0,00001 à 1 % en poids d'un ou de plusieurs composés du groupe comportant
a. le 4,7-diméthoxy-5-(prop-2-enyl)-2H(1,3-benzodioxole) et/ou
b. le 4,5-diméthoxy-6-(prop-2-enyl)-2H(1,3-benzodioxole) et/ou
c. des mélanges de ceux-ci,
b) de 0,001 à 1 % en poids d'un ou de plusieurs composés du groupe comortant
d. le 2-méthoxy-4-(prop-2-ényl)-phénol et/ou
e. le 2-méthoxy-4-(prop-1-ényl)-phénol et/ou
f. des mélanges de ceux-ci,
c) de 10 à 50 % en poids d'un ou de plusieurs alcools polyvalents du groupe comportant le sorbitol et/ou le glycérol et/ou le 1,2-propylène-glycol ou leurs mélanges,
à condition que l'agent de soin et de nettoyage buccodentaire comprenne de 0,05 à 0,4 % en poids de 2-méthoxy-4-(prop-1-ényl)-phénol

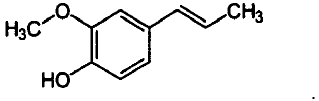

2. Agent de soin et de nettoyage buccodentaire selon la revendication 1, **caractérisé en ce qu'**il contient de 0,000025 à 0,5 % en poids, de préférence de 0,00005 à 0,25 % en poids, de manière particulièrement préférée de 0,0001 à 0,1 % en poids, plus préférablement de 0,0005 à 0,05 % en poids, et en particulier de 0,001 à 0,01 % en poids de 4,7-diméthoxy-5-(prop-2-ényl)-2H(1,3-benzodioxole).

3. Agent de soin et de nettoyage buccodentaire selon la revendication 1 ou 2, **caractérisé en ce qu'**il contient de 0,0025 à 0,5% en poids, de préférence de 0,05 à 0,4% en poids, de manière particulièrement préférée de 0,01 à 0,3% en poids, plus préférablement de 0,05 à 0,2% en poids et en particulier de 0,075 à 0,1 % en poids de 2-méthoxy-4-(prop-2-ényl)phénol.

4. Agent de soin et de nettoyage buccodentaire selon l'une des revendications 1 à 3, **caractérisé en ce qu'**il contient de 0,00004 à 0,25 % en poids, de manière particulièrement préférée de 0,0001 à 0,1 % en poids, plus préférablement de 0,00025 à 0,05 % en poids et en particulier de 0,001 à 0,01% en poids de (4,7-diméthoxy-5-(prop-2-ényl)-2H(1,3-benzodioxole) et de 0,001 à 0,4 % en poids, de préférence de 0,005 à 0,3 % en poids, de manière particulièrement préférée de 0,01 à 0,25 % en poids, plus préférablement de 0,05 à 0,15 % en poids et en particulier de 0,05 % à 0,1% en poids de 2-méthoxy-4-(prop-2-ényl)-phénol.

5. Agent de soin et de nettoyage buccodentaire selon l'une des revendications 1 à 4, **caractérisé en ce que** le rapport en poids du ou des composés (b) au ou aux composés (a) est ≥ 1:1, de préférence de ≥ 1,2:1, de manière particulièrement préférée ≥ 1,4:1 et en particulier de ≥ 1,5:1.

6. Agent de soin et de nettoyage buccodentaire selon l'une des revendications 1 à 5, **caractérisé en ce qu'**il contient de 0,001 à 5 % en poids, de préférence de 0,02 à 2,5 % en poids, de manière particulièrement préférée de 0,03 à 2,0 % en poids, plus préférablement de 0,04 à 1,5 % en poids, encore plus préférablement 0,05 à 1,0 % en poids, encore plus préférablement de 0,06 à 0,5 % en poids, et en particulier de 0,07 à 0,25 % en poids d'au moins un sel de zinc.

7. Agent de soin et de nettoyage buccodentaire selon l'une des revendications 1 à 6, **caractérisé en ce qu'**il contient du sulfate de zinc, de préférence du sulfate de zinc heptahydraté.

8. Agent de soin et de nettoyage buccodentaire selon l'une des revendications 6 à 7, **caractérisé en ce que** le rapport en poids du ou des sels de zinc au ou aux composés (a) et (b) est ≥ 1:1, de préférence ≥ 1,2:1, de manière particulièrement préférée ≥ 1,4:1 et en particulier ≥ 1,5:1.

9. Agent de soin et de nettoyage buccodentaire selon l'une des revendications 1 à 8, **caractérisé en ce qu'**il contient de 0,001 à 5 % en poids, de préférence de 0,005 à 2,5% en poids, de manière particulièrement préférée de 0,01 à 1% en poids, de manière extraordinairement préférée de 0,025 à 0,8 % et en particulier de 0,05 à 0,8 % en poids de chlorure de cétylpyridinium et/ou de chlorure de laurylpyridinium et/ou de chlorure de 1-hexadécylpyridinium ou de leurs mélanges.

10. Agent de soin et de nettoyage buccodentaire selon l'une des revendications 1 à 9, **caractérisé en ce qu'**il contient, par rapport à son poids total, de 0,05 à 5,0 % en poids, de préférence de 0,1 à 3,5 % en poids, de manière particulièrement préférée de 0,2 à 2,5 % en poids et en particulier de 0,5 à 2,0 % en poids d'un ou de plusieurs tensioactifs amphotères, de préférence de cocamidopropylbétaïne.

11. Agent de soin et de nettoyage buccodentaire selon l'une des revendications 1 à 10, **caractérisé en ce qu'**il contient, par rapport à son poids total, de 0,05 à 5,0 % en poids, de préférence de 0,1 à 3,5 % en poids, de façon particulièrement préférée de 0,2 à 2,5 % en poids, en particulier de 0,5 à 2,0 % en poids d'un ou de plusieurs tensioactifs anioniques, de préférence du laurylsulfate de sodium.
